Europäisches Patentamt

European Patent Office

Office européen des brevets

(11) Publication number: **0 234 088 B1**

## (12) EUROPEAN PATENT SPECIFICATION

(45) Date of publication of patent specification: **19.02.92** (51) Int. Cl.5: **C07D 201/04**

(21) Application number: **86306672.6**

(22) Date of filing: **28.08.86**

The file contains technical information submitted after the application was filed and not included in this specification

(54) Production of E-caprolactam.

(30) Priority: **28.08.85 JP 190593/85**
**25.02.86 JP 40927/86**

(43) Date of publication of application:
**02.09.87 Bulletin 87/36**

(45) Publication of the grant of the patent:
**19.02.92 Bulletin 92/08**

(84) Designated Contracting States:
**BE DE GB IT NL**

(56) References cited:
**EP-A- 0 168 978**
**US-A- 3 503 958**
**US-A- 4 359 421**
**US-A- 4 472 516**

**CHEMICAL ABSTRACTS, vol.99, no.13, page 2, 26.09.83. (Columbus, Ohio, USA) & DOKL. AKAD. NANK BSSR, 1983, 27(8) 723-64**

**CHEMICAL ABSTRACTS, vol.108, no.13, page 573, 28.03.88. (Columbus, Ohio, USA).**

**JOURNAL OF CATALYSIS, vol.6, 1966, pages 245-252, Landis Venuto.**

**JOURNAL OF CATALYSIS, vol.61, 1980, pages 390-396, Olson et al.**

(73) Proprietor: **SUMITOMO CHEMICAL COMPANY, LIMITED**
**Kitahama 4-chome 5-33**
**Chuo-ku Osaka 541(JP)**

(72) Inventor: **Sato, Hiroshi**
**6-3, Funakicho**
**Ibaraki-shi Osaka 567(JP)**
Inventor: **Hirose, Kenichi**
**5-5-12-108, Senrioka**
**Settsu-shi Osaka 564(JP)**
Inventor: **Ishii, NorioöSumitomo Chemical Co. Ltd.**
**Seikiryo 20-1, Hoshigoecho**
**Niihama-shi Ehime 792(JP)**
Inventor: **Umada, Youichi**
**13-5, Nishimachi**
**Takatsuki-shi Osaka 569(JP)**

(74) Representative: **Sheard, Andrew Gregory et al**
**Kilburn & Strode 30, John Street**
**London WC1N 2DD(GB)**

Rank Xerox (UK) Business Services

**Description**

This invention relates to the production of $\epsilon$-caprolactam and, more particularly, to the use of a specific crystalline alumino-silicate catalyst in the production of $\epsilon$-caprolactam from cyclohexanone oxime.

$\epsilon$-caprolactam is an important material for nylon and the like. One of the processes for preparing caprolactam is a liquid-phase catalytic rearrangement of cyclohexanone oxime in the presence of sulphuric acid. Alternatively, gas-phase catalytic rearrangements in the presence of solid acid have been proposed, e.g., boric acid compounds (Japanese published unexamined patent appliation Nos. 37686/1978 and 12125/1971), silica-alumina (British patent No. 881927), solid phosphoric acid (British patent No. 881926), mixed metal oxides (Journal of the Chemical Society of Japan, No. 1, 77, 1977), Y-zeolite (Journal of Catalysis, 6, 247, 1966) and crystalline alumino-silicate (Japanese published unexamined patent application No. 139062/1982).

When sulphuric acid is used, problems encountered include: handling a large amount of fuming sulphuric acid; the by-production of a large amount of ammonium sulphate; and corrosion of the apparatus caused by the fuming sulphuric acid. There are not such problems in the alternative processes above in which solid acid is used. However, they have the other difficulties such as the reaction selectivity for $\epsilon$-caprolactam, the life of the catalyst, the production yield per catalyst, the conversion rate of the oxime etc. For instance, Japanese published unexamined patent application No. 139062/1982, where crystalline alumino-silicate e.g. ZSM-5 having 40 - 60 of Si/Al atomic ratio is used, discloses no selectivity for $\epsilon$-caprolactam; but the conversion rate of cyclohexanone oxime is said to be quantitative in cases where the weight hourly space velocity (hereinafter referred to as "WHSV") is as low as about 2.5 hr$^{-1}$ and the life of the catalyst is as short as 15 - 20 hours. The present inventors have repeated the process of the Japanese published unexamined patent application mentioned above to confirm the shortness of life of the catalyst and the low selectivity for $\epsilon$-caprolactam, particularly the facts that the life of the catalyst is too short and the selectivity under commercial WHSVs (for example, about 10 hr$^{-1}$ or higher) is too low.

The present inventors have studied the production of $\epsilon$-caprolactam using a crystalline alumino-silicate catalyst having a constraint index of 1 - 12. It was found that alumino-silicates having particular Si/Al atomic ratios and particular acid functionalities of the external surface greatly improve the conversion rate of the oxime, the selectivity for the lactam, the life of the catalyst, and productivity.

USP 4359421 describes a number of ZSM zeolite catalysts having Si/Al ratios in the range 36 to 56 for converting cyclohexanone oxime to $\epsilon$-caprolactam. At the higher Si/Al ratio the time for conversion to drop to 40% is eighteen hours, while at the lower ratio the time for the conversion to drop to 40% is longer, namely nineteen hours.

According to the present invention, in the production of $\epsilon$-caprolactam by gas-phase catalytic rearrangement of cyclohexanone oxime in the presence of crystalline alumino-silicate having a constraint index of 1 - 12, an improvement in the crystalline catalyst is provided when the Si/Al atomic ratio is not smaller than 500 and an acid functionality of the external surface is not greater than 5 micro equivalent/g. It would not be expected that an alumino-silicate catalyst having such a high silica content as well as such a low acidity (as defined above with respect to the Si/Al atomic ratio and to the acid functionality of the external surface) would have such a high catalytic performance (in terms of oxime conversion rate), in view of the teaching that such a catalyst is hardly workable (Journal of Catal. 61, 393, 1980). In addition Chem. Abs. 99:105.742r (1983) teaches that for an HNa Y zeolite catalyst when the mole ratio of $SiO_2/Al_2O_3$ increases from 4.9 to 6.41 the yield of $\epsilon$-caprolactam (from cyclohexanone oxime) rises to 75.3% but when the mole ratio is increased further to 16.5 the yield falls to 14%. Furthermore, the present catalyst greatly improves selectivity for lactam, catalyst life and productivity, when compared with a catalyst having an Si/Al atomic ratio of not higher than 100, which will be familiar to those skilled in the art.

The constraint index, indicates the ability, by the pore structure of the crystalline alumino-silicate, to control access of molecules having a larger sectional area than n-hexane and is defined by the formula given below:

$$\text{Constraint index} = \frac{\log_{10}(\text{remaining n-hexane})}{\log_{10}(\text{remaining 3-methylpentane})}$$

In outline, a mixture of n-hexane and 3-methylpentane, which have different effective molecular diameters, is brought into contact under specified conditions with an alumino-silicate catalyst to cause a

2

cracking reaction; the constraint index is calculated on the basis of the reaction ratio. Values vary somewhat depending on experimental conditions. An average value is taken, after the experiment is done under various conditions. Details are given in Japanese published unexamined patent application No. 133223/1981.

Crystalline alumino-silicate having a constraint index of 1 - 12 includes: "Nu zeolite" and "EU zeolite" manufactured by ICC, (Catalysis Reviews-Science & Engineering 27, 461, 1985); "ZBM zeolite" manufactured by BASF (West Germany Patent Nos. 2830787 (1980) and 3006471 (1981)); "TPZ zeolite" manufactured by Teijin Yuka Co. Ltd., (Japanese published unexamined patent application Nos. 95281/1982 and 149819/1982) in addition to "ZSM zeolite" manufactured by Mobile Oil Co. and "Silicalite" manufactured by U.C.C. Examples are "ZSM-5" [Constraint index: 8.3 (hereinafter, the term "Constraint index" is omitted), Japanese published unexamined patent application No. 10064/1971], "ZSM-11" (8.7, Japanese published examined patent application No. 23280/1978), "ZSM-12" (2, Japanese published examined patent application No. 16079/1977), "ZSM-23" (9.1, Japanese published examined patent application No. 149900/1976), "ZSM-35" (4.5, Japanese published unexamined patent application No. 144500/1978), "ZSM-38" (2, U.S. Patent No. 4046859), "ZSM-48" (3.4, Japanese published unexamined patent application No. 133223/1981) and "Silicalite" (8.5, U.S. Patent No. 4061724). X-ray patterns and preparative processes therefor are disclosed in the respective items of literature. "ZSM-5" and "Silicalite" are preferred.

The present catalyst additionally satisfies two more parameters, i.e., an Si/Al atomic ratio of 500 or more, and an acid amount or functionality of the external surface of 5 micro equivalent/g or less, preferably 2 micro equivalent/g or less. These limits are critical. The Si/Al atomic ratio is calculated on the basis of atomic adsorption spectroscopy after crystallinity has been measured by, for example, X-ray, and then an exact elemental analysis of Si and Al in the crystal skeleton is made. Another approach for the Si/Al atomic ratio in the skeleton is from the $^{29}$Si signal in MAS-NMR spectrum. The acid functionality of the external surface is measured by the amount in equivalents of 4-methylquinoline adsorbed (Journal of Petroleum Chemical Society of Japan, 25, 69, 1982; Journal of Catal. 58, 114, 1979).

Generally speaking, particularly in a high silica region as in the present invention, the crystallinity as well as the crystal growth increase while the external surface area decreases as the Si/Al atomic ratio increases. The greater the external surface area is, the better the catalytic activity and selectivity are. 5 $m^2$/g or more is preferred. The external surface area is observed by a pore-filling process, i.e., pores in the crystal are filled up with organic or inorganic molecules and then the necessary surface area is observed by the BET method on the basis of the amount of nitrogen or krypton adsorbed onto the external surface. The molecules to fill up the pores are organic such as butane, hexane, benzene and the like, water (7th and 8th Seminars on catalysts, Catalyst Society, Japan, 1984 and 1985) and organic amines or a tetra-alkylammonium cation used as a crystallization-controlling agent in hydrothermal synthesis. (Hydrothermal synthesis of crystalline alumino-silicate in which constraint index is 1 - 12 and Si/Al atomic ratio is 500 or more, can be effected in the presence of, usually, organic amines or tetra-alkylammonium cation as a crystallization-controlling agent which acts as a filler for pores just when the synthesis is finished.) The BET surface area of zeolite is the external surface area, the zeolite having been dried at a temperature up to 120°C immediately after the synthesis is finished.

The crystalline alumino-silicate catalyst having a high silica content and low acidity mentioned above may be selected from crystalline alumino-silicates prepared by processes disclosed, for example, in Japanese published unexamined patent application No. 164617/1984 or U.S. Patent No. 4061724. A Si-source for preparing the compounds should be as pure as possible having a very small amount of Al impurity, e.g., tetra-alkyl ortho-silicate, aerogel, colloidal silica, sodium silicate (JIS Grade No. 3 of water glass).

Alumino-silicate obtained from hydrothermal synthesis should be calcined in air until any organic amine cation is removed, then subjected to ion-exchange with aqueous ammonium chloride solution or dilute aqueous hydrochloric acid solution and then calcined again until is is converted to the $H^+$ form, since the silicate usually contains an organic amine cation used as a crystallization-controlling agent and alkali metal cations such as $Na^+$, $K^+$. Alternatively, a corresponding polyvalent metal ion form may be used in place of the $H^+$ form, in which case ion-exchange is effected by use of aqueous alkaline earth metal solution containing, for example, $Ca^{2+}$, $Mg^{2+}$, $Sr^{2+}$ and $Ba^{2+}$, or aqueous lanthanide series solution containig, for example, $La^{2+}$ and $Ce^{3+}$ in place of the aqueous ammonium chloride or dilute hydrochloric acid solution.

Gas phase catalytic reactions can be carried out in a fixed-bed or flow-layer operations. Cyclohexanone oxime, the starting compound, is vaporized in a vaporizer and then is brought in the gas form into contact with the catalyst. The cyclohexanone oxime may be fed in alone, but is preferably in the form of a solution in benzene or toluene to the vaporizer. In the latter case, the solution may be carried by an inert carrier gas such as $N_2$, $CO_2$ etc. $CO_2$ gas is preferred since the selectivity for lactam is improved.

3

The reaction temperture is usually 250 - 500°C, preferably 300 - 450°C. The feeding speed (WHSV) is generally 0.1 - 100 $hr^{-1}$, preferably 1 - 50 $hr^{-1}$, more preferably 5 - 40 $hr^{-1}$.

Regeneration of a catalyst whose activity decreases after long use is effected by calcining at 450 - 550°C in an air stream.

Isolation of $\epsilon$-caprolactam is effected by, for example, cooling and condensing the reaction gas and then distilling or recrystallizing.

In accordance with the present process, conversion rate of cyclohexanone oxime and selectivity of $\epsilon$-caprolactam are greatly improved, the amount of carbon deposition on a catalyst is very small, catalyst life is greatly prolonged and the yield of $\epsilon$-caprolactam is generally high for a long period of time. Furthermore, a high degree of productivity per catalyst is attainable with a high WHSV. Catalyst activity can easily be recovered by calcining a used catalyst in air. Repeated use of the catalyst is possible.

Reference Example 1

(Synthesis of high silica H.ZSM-5)

"Aerozil" (trade name, manufactured by Japan Aerozil Co. Ltd., Al $\leq$ 8.8 ppm, 26 g), colloidal silica (manufactured by Shokubai Kasei, Co. Ltd., Japan, ST-30, $SiO_2$ = 30%) (200 g), distilled water (230 g) and an aqueous solution (250 g) containing tetra-n-propylammonium bromide (34 g) were charged in an autoclave (1.5ℓ) made of SUS, and the content was vigorously stirred. An aqueous solution (100 g) containing sodium hydroxide (7.4 g) was charged all at once and the mixture was vigorously stirred for 30 min. The autoclave was tightly sealed, and then dipped in an oil bath until the inner temperature reached 190°C. Stirring (400 r.p.m.) was continued for 72 hours keeping the temperature as above. Pressure within the autoclave reached 16 $Kg/cm^2$ from 14 $Kg/cm^2$. The pH at the end of the hydrothermal synthesis was 11.8. White solid product was separated by filtration. The product was continuously washed with distilled water until the filtrate pH reached about 7. The product was dried at 120°C for 16 hours. The BET crystal surface at that stage was observed by nitrogen gas adsorption method to have an external surface area of 1.4 $m^2/g$.

The dried crystals were calcined in an air stream at 500 - 530°C for 4 hours to obtain 80 g of powdery white crystals.

By powder X ray diffraction assay, ZSM-5 was identified.

Si/Al atomic ratio = 2,950 (atomic absorption spectroscopy assay).

To the crystals (10 g) was added 5% aqueous $NH_4Cl$ solution (100 g) and then the mixture was subjected to ion-exchange at 50 - 60°C for one hour before filtration. The ion-exchange treatment was repeated four times. Crystals were washed with distilled water until no $Cl^-$ was detected. Crystals were dried at 120°C for 16 hours. Crystals ($NH_4$ form) were shaped to particles (24 - 48 mesh in size) and calcined at 500°C for 4 hours to obtain ZSM-5 (H form, hereinafter referred to as "H·ZSM-5").

Surface acidity (pKa) = -3 (an indicator assay).

Amount of 4-methylquinoline (which is referred to as 4 MQ hereinafter) adsorbed at 350°C = 0.22$\mu$ equivalent/g.

Example 1 (A test under high WHSV)

H·ZSM-5 (0.3 g, 0.5 ml, 24 - 48 mesh in size), a catalyst, prepared in Reference Example 1 above was packed in a reactor made of quartz (32 cm long, 1 cm inner diameter) and $N_2$ gas flowed at 350°C for one hour. After the pre-treatment, 8 wt.% cyclohexanone/benzene solution was fed (WHSV = 38.5 $hr^{-1}$) from a vaporizer. The temperature of the catalyst layer was 350°C. Reaction product was trapped and collected under water-cooling. Gas-chromatography [ column: 20% silicone SE-30/chromosorb AW-DMCS (60/80 M) 2 m : glass column, internal standard pseudocumene] gave the result shown in Table 1.

4

| time elapsed (hr) | cyclohexanone oxime conversion (%) | ε-caprolactam | |
| --- | --- | --- | --- |
| | | yield (%) | selectivity (%) |
| 1.3 | 73.8 | 53.2 | 72.2 |
| 2.3 | 49.4 | 36.5 | 73.5 |
| 3.3 | 37.2 | 27.4 | 73.7 |
| 4.3 | 30.3 | 22.8 | 75.2 |
| 5.3 | 26.3 | 19.6 | 74.5 |
| 6.3 | 24.4 | 17.4 | 71.1 |

Example 2 (A test under high WHSV)

Example 1 was repeated except that the catalyst used was "Silicalite" (0.3 g, 0.5 ml, 24 - 48 mesh in size) prepared by Reference Example 2 in place of the H•ZSM-5.

The result is shown in Table 2.

| time elapsed (hr) | cyclohexanone oxime conversion (%) | ε-caprolactam | |
| --- | --- | --- | --- |
| | | yield (%) | selectivity (%) |
| 1.3 | 65.4 | 44.0 | 67.3 |
| 2.3 | 55.9 | 41.2 | 73.7 |
| 3.3 | 53.9 | 38.4 | 71.3 |
| 4.3 | 50.7 | 35.7 | 70.5 |
| 5.3 | 46.7 | 34.0 | 72.9 |
| 6.3 | 45.5 | 31.8 | 69.8 |
| 7.3 | 43.5 | 31.1 | 71.5 |

Reference Example 2

(Synthesis of highly pure "Silicalite")

"Silicalite" was prepared according to US patent No. 4061724. "Aerozil" (manufactured by Japan Aerozil Co. Ltd., Al ≤ 8.8 ppm, 70 g), distilled water (600 g) and aqueous solution (156 g) containing tetra-n-propylammonium bromide (36 g) were charged in an autoclave (1.5 ℓ) made of SUS, and the content was vigorously stirred. An aqueous solution (60 g) containing sodium hydroxide (7.8 g) was added all at once and the mixture was vigorously stirred for 30 min. The autoclave was tightly sealed and dipped in an oil bath until the inner temperature reached 190 °C. Stirring (400 r.p.m.) was continued for 72 hours at that temperature until pressure in the autoclave increased from 14 Kg/cm$^2$ to 16 Kg/cm$^2$. As in Reference Example 1, the steps of calcining, ion exchange with aqueous $NH_4Cl$ solution, and re-calcining were effected to obtain "Silicalite".

Si/Al atomic ratio = 25,000
4-MQ adsorption amount = 0.01μ equivalent/g
External surface area = 1.5 m$^2$/g,
Surface acidity = from - 3.0 to + 4.8

Reference Example 3

(Synthesis of high silica H·ZSM-5)

To an autoclave (1.5 ℓ) made of SUS were charged Si(OEt)$_4$ (90 g, manufactured by Hani Chemical Co. Ltd, Japan) containing aqueous 20 - 25% tetra-n-propylammonium hydroxide solution (130 g), ethanol (60 g) and $H_2O$ (100 g). Colloidal silica (SI-30) (200 g, manufactured by Shokubai Kasei Co. Ltd., Japan) was added to the content in the autoclave all at once under vigorous stirring. The vigorous stirring was continued for 30 min. after the addition. The autoclave was tightly sealed and dipped in an oil bath until the temperature reached 160 °C. Stirring (400 r.p.m.) was continued for 120 hours at that temperature until the pressure reached 9.5 - 11 Kg/cm$^2$. As in Reference Example 1, the steps of drying, calcining, ion exchange with aqueous $NH_4Cl$ solution and re-calcining were effected to obtain H·ZSM-5.

Si/Al atomic ratio = 2410
4 MQ adsorption amount ≒ 0μ equivalent/g
Surface acidity pKa = - 3.0
External surface area = 3.0 m$^2$/g

Example 3 (A test under high WHSV)

Example 1 was repeated to effect rearrangement of cyclohexanone oxime except that the catalyst used was H·ZSM-5 prepared by Reference Example 3, in place of the H·ZSM-5.

The result is shown in Table 3.

## Table 3

| time elapsed (hr) | cyclohexanone oxime conversion (%) | ε-caprolactam | |
|---|---|---|---|
| | | yield (%) | selectivity (%) |
| 1.3 | 70.7 | 43.4 | 61.4 |
| 2.3 | 53.3 | 36.1 | 67.8 |
| 3.3 | 44.6 | 31.4 | 70.3 |
| 4.3 | 39.0 | 27.0 | 69.4 |
| 5.3 | 37.0 | 23.7 | 64.1 |
| 6.3 | 34.1 | 21.0 | 61.4 |

Reference Example 4 (Synthesis of high silica H·ZSM-5)

ZSM-5 was synthesized according to Japanese published unexamined patent application No. 164617/1984. That is, to an autoclave (1.5 ℓ) made of stainless-steel, were charged tetra-ethyl ortho-silicate [Si(OEt)₄, 100g, Al < 10 ppm], aqueous 10 % tetra-n-propylammonium hydrochloride solution (224 g) and ethanol (60 g) and the content was stirred well. To this was added aqueous aluminium sulphate solution (48 g) (Al₂(SO₄)₃·16H₂O, 62 mg/48 g of water) and the mixture (pH 12.4) was stirred vigorously for 30 min. The autoclave was tightly sealed and dipped in an oil bath until temperature reached 160°C. Hydrothermal synthesis was conducted for 120 hours at that temperature with stirring (at least 400 r.p.m.), until the pressure reached 14 Kg/cm² from 12 Kg/cm² and the pH was 11.7. A white solid product was filtered and continuously washed with distilled water until pH of filtrate was about 7. The product was dried at 120°C for 16 hours. The BET crystal surface area was 4.9 m²/g in terms of the external surface area (nitrogen gas adsorption assay). The dried crystals were calcined at 500 - 530°C for 4 hours in air stream to obtain powdery white crystals (27 g). These were identified as ZSM-5 (powder X ray diffraction).
Si/Al atomic ratio = 2,290 (atomic absorptiometry)

To 10 g of these crystals was added aqueous 5% NH₄Cl solution (100 g) to carry out ion-exchange at 50 - 60°C for one hour, before filtration. The ion-exchange step was repeated four times. Crystals were washed with distilled water until no Cl⁻ was detected, before they were dried at 120°C for 16 hours. Crystals (NH₄ form) were shaped to particles (24 - 48 mesh in size) and calcined at 500°C for 4 hours to convert to H·ZSM-5.
Surface acidity pKa = -3 (an indicator assay)

4-MQ adsorption amount at 350°C = 2.23 μ equivalent/g.

Example 4

Example 1 was repeated except that the catalyst used was H·ZSM-5 prepared in Reference Example 4 in place of the H·ZSM-5.

The result is shown in Table 4.

## Table 4

| time elapsed (hr) | cyclohexanone oxime conversion (%) | ε-caprolactam | |
|---|---|---|---|
| | | yield (%) | selectivity (%) |
| 1.3 | 72.3 | 45.3 | 62.7 |
| 2.3 | 63.4 | 38.3 | 60.4 |
| 3.3 | 58.4 | 35.1 | 60.1 |
| 4.3 | 54.8 | 33.7 | 61.5 |
| 5.3 | 52.4 | 32.2 | 61.4 |
| 6.3 | 50.9 | 29.8 | 58.6 |

Reference Example 5

(Synthesis of high silica H·ZSM-5)

Reference Example 4 was repeated to effect hydrothermal synthesis except that amount of aluminium sulphate was changed as in Table 5. After calcining, ion-exchange with NH₄Cl and re-calcining, samples of H.ZSM-5 having Si/Al atomic ratios as in Table 6 were obtained.

Table 5

| No. | Si(OEt)$_4$ | Al$_2$(SO$_4$)$_3$. 16 H$_2$O(g) | pH when charged | pH when finished | Si/Al ratio when charged |
|-----|-------------|----------------------------------|-----------------|------------------|--------------------------|
| 1 | 100 | 0.078 | 13.0 | 11.8 | 2,000 |
| 2 | 100 | 0.031 | 12.8 | 11.3 | 5,000 |
| 3 | 100 | 0.125 | 12.6 | 12.2 | 1,250 |

Table 6

| No. | Si/Al (atomic ratio) | 4MQ adsorption amount ($\mu$ equivalent/g) | Surface acidity (pKa) | External surface area (m$^2$/g) |
|-----|----------------------|--------------------------------------------|-----------------------|--------------------------------|
| 1 | 2,017 | 0.84 | - 3.0 | 9.2 |
| 2 | 3,930 | 0.36 | - 3.0 | 5.7 |
| 3 | 1,160 | 0.06 | - 3.0 | 5.8 |

Examples 5 - 7 (Tests under high WHSV)

Example 1 was repeated except that catalysts used were H•ZSM-5 Nos. 1 - 3 prepared in Reference example 5, in place of the H.ZSM-5.

Results are shown in Tables 7 - 9.

Table 7 (No. 1 catalyst)

| time elapsed (hr) | cyclohexanone oxime conversion (%) | ε-caprolactam | |
| --- | --- | --- | --- |
| | | yield (%) | selectivity (%) |
| 2.3 | 99.3 | 71.8 | 72.3 |
| 3.3 | 98.1 | 71.7 | 73.1 |
| 4.3 | 96.2 | 70 6 | 73.4 |
| 5.3 | 94.2 | 67.5 | 71.6 |
| 6.3 | 91.7 | 65.1 | 71.0 |

Table 8 (No. 2 catalyst)

| time elapsed (hr) | cyclohexanone oxime conversion (%) | ε-caprolactam | |
| --- | --- | --- | --- |
| | | yield (%) | selectivity (%) |
| 1.3 | 98.7 | 65.1 | 66.0 |
| 2.3 | 93.3 | 66.7 | 71.4 |
| 3.3 | 85.5 | 59.5 | 69.6 |
| 4.3 | 78.3 | 56.2 | 71.8 |
| 5.3 | 71.2 | 52.2 | 73.3 |
| 6.3 | 64.7 | 48.3 | 74.5 |
| 7.3 | 60.3 | 42.8 | 71.0 |

9

**Table 9 (No. 3 catalyst)**

| time elapsed (hr) | cyclohexanone oxime conversion (%) | ε-caprolactam | |
|---|---|---|---|
| | | yield (%) | selectivity (%) |
| 1.3 | 98.4 | 67.9 | 69.1 |
| 2.3 | 93.8 | 67.6 | 72.1 |
| 3.3 | 88.0 | 64.5 | 73.3 |
| 4.3 | 81.8 | 60.1 | 73.5 |
| 5.3 | 74.9 | 59.4 | 79.3 |
| 6.3 | 70.5 | 53.2 | 75.6 |
| 7.3 | 67.3 | 48.3 | 71.7 |

Example 8 (A test under high WHSV)

Example 5 was repeated except that the catalyst used therein was calcined in a reactor at 500°C for 3 hours in air stream, in place of the H·ZSM-5.

Result is shown in Table 10.

**Table 10**

| time elapsed (hr) | cyclohexanone oxime conversion (%) | ε-caprolactam | |
|---|---|---|---|
| | | yield (%) | selectivity (%) |
| 1.3 | 100 | 71.0 | 71.0 |
| 2.3 | 99.5 | 71.6 | 72.0 |
| 3.3 | 98.0 | 71.1 | 72.5 |
| 4.3 | 96.0 | 69.0 | 72.0 |
| 5.3 | 94.1 | 67.6 | 71.8 |
| 6.3 | 92.0 | 66.5 | 72.3 |

Reference Example 6

(Synthesis of metal ion-exchanged ZSM-5)

Samples of ZSM-5 ($NH_4$ form) obtained in Reference Example 5, No. 1 (4 g each) were subjected four times, at 90°C for one hour each, to ion-exchange treatment with aqueous 5 % solution (50 ml each) containing metal salts (MXn X = $Cl^-$ or $OAc^-$) shown in Table 11. After washing with water, drying and calcining, metal ion-exchanged ZSM-5 samples were obtained.

10

Table 11

| Run No. | Metal salts | M·ZSM-5 |
|---------|-------------|---------|
| 1 | Ca(OAc)$_2$ | Ca·ZSM-5 |
| 2 | Sr(OAc)$_2$ | Sr·ZSM-5 |
| 3 | LaCl$_3$ | La·ZSM-5 |

Examples 9 - 11 (Tests under high WHSV)

Example 1 was repeated except that samples of metal ion-exchanged ZSM-5 prepared in Reference Example 6 were used in place of H.ZSM-5 in Example 5.

The results are shown in Tables 12 - 14.

Table 12 (Ca·ZSM-5)

| time elapsed (hr) | cyclohexanone oxime conversion (%) | ε-caprolactam | |
|---|---|---|---|
| | | yield (%) | selectivity (%) |
| 1.3 | 100 | 72.0 | 72.0 |
| 2.3 | 99.0 | 73.3 | 74.0 |
| 3.3 | 98.1 | 70.9 | 72.3 |
| 4.3 | 96.0 | 67.7 | 70.5 |
| 5.3 | 94.0 | 69.0 | 73.0 |
| 6.3 | 90.2 | 65.8 | 73.0 |

### Table 13 (Sr·ZSM-5)

| time elapsed (hr) | cyclohexanone oxime conversion (%) | ε-caprolactam | |
|---|---|---|---|
| | | yield (%) | selectivity (%) |
| 1.3 | 100 | 70.3 | 70.3 |
| 2.3 | 100 | 70.5 | 70.5 |
| 3.3 | 100 | 71.0 | 71.0 |
| 4.3 | 99.3 | 72.3 | 72.8 |
| 5.3 | 98.1 | 70.6 | 72.0 |
| 6.3 | 96.0 | 67.8 | 70.6 |

### Table 14 (La·ZSM-5)

| time elapsed (hr) | cyclohexanone oxime conversion (%) | ε-caprolactam | |
|---|---|---|---|
| | | yield (%) | selectivity (%) |
| 1.3 | 99.0 | 69.3 | 70.0 |
| 2.3 | 98.1 | 69.8 | 71.2 |
| 3.3 | 97.0 | 69.2 | 71.3 |
| 4.3 | 96.2 | 69.6 | 72.4 |
| 5.3 | 94.0 | 67.2 | 71.5 |
| 6.3 | 91.0 | 64.3 | 70.7 |

Reference Example 7

Reference Example 4 was repeated except that the hydrothermal synthesis was effected using aluminium sulphate and at the temperature shown in Table 15 to obtain H·ZSM-5 as shown in Table 16.

Table 15

| No. | $Al_2(SO_4)_3 \cdot 16H_2O$ (mg) | temperature |
|---|---|---|
| 1 | 98 | 105 |
| 2 | 70 | 160 |
| 3 | 54 | 130 |
| 4 | 0 | 105 |

# EP 0 234 088 B1

Table 16

| No. | Si/Al atomic ratio | 4MQ adsorption amount ($\mu$ equivalent/g) | Surface acidity (pKa) | External surface area (m²/g) |
|---|---|---|---|---|
| 1 | 1,600 | 3.92 | - 3.0 | 10.3 |
| 2 | 2,240 | 2.01 | - 3.0 | 7.3 |
| 3 | 2,900 | 0.35 | - 3.0 | 8.7 |
| 4 | 27,000 | * 1 | * 2 | 11.7 |

Notes: *1

zero

*2 an indicator (pKa = - 3, dicinnamylacetone) is slightly coloured

Examples 12 - 14 (Tests under high WHSV)

Example 1 was repeated except that H.ZSM-5, Nos. 1 - 3 prepared in Reference Example 7 were employed, respectively, in place of the H•ZSM-5.

The results are shown in Tables 17 - 19.

## Table 17 (No. 1 catalyst of Reference Example 7)

| time elapsed (hr) | cyclohexanone oxime conversion (%) | $\varepsilon$-caprolactam | |
|---|---|---|---|
| | | yield (%) | selectivity (%) |
| 1.3 | 99.0 | 72.1 | 72.9 |
| 2.3 | 97.7 | 74.6 | 76.4 |
| 3.3 | 96.1 | 77.4 | 80.5 |
| 4.3 | 94.1 | 77.7 | 82.6 |
| 5.3 | 92.5 | 74.3 | 80.3 |
| 6.3 | 90.9 | 73.0 | 80.0 |

13

Table 18 (No. 2 catalyst of Reference Example 7)

| time elapsed (hr) | cyclohexanone oxime conversion (%) | ε-caprolactam | |
|---|---|---|---|
| | | yield (%) | selectivity (%) |
| 1.3 | 99.5 | 83.4 | 84.8 |
| 2.3 | 98.3 | 82.7 | 84.1 |
| 3.3 | 98.0 | 82.0 | 83.6 |
| 4.3 | 96.9 | 83.4 | 86.1 |
| 5.3 | 95.6 | 81.1 | 84.9 |
| 6.3 | 93.7 | 81.7 | 87.2 |
| 7.3 | 92.2 | 79.8 | 86.6 |

Table 19 (No. 3 catalyst of Reference Example 7)

| time elapsed (hr) | cyclohexanone oxime conversion (%) | ε-caprolactam | |
|---|---|---|---|
| | | yield (%) | selectivity (%) |
| 1.3 | 100 | 81.6 | 81.6 |
| 2.3 | 99.5 | 82.0 | 82.4 |
| 3.3 | 97.4 | 82.5 | 84.7 |
| 4.3 | 95.5 | 81.3 | 83.5 |
| 5.3 | 93.2 | 80.0 | 85.8 |
| 6.3 | 92.0 | 80.3 | 87.3 |

Example 15 (A test under high WHSV)

Example 12 was repeated except the catalyst used therein was calcined in a reactor at 500°C for 3 hours in air stream, in place of the H·ZSM-5. The result is shown in Table 20.

14

## Table 20

| time elapsed (hr) | cyclohexanone oxime conversion (%) | ε-caprolactam | |
|---|---|---|---|
| | | yield (%) | selectivity (%) |
| 1.3 | 99.0 | 72.1 | 72.9 |
| 2.3 | 97.6 | 74.5 | 76.3 |
| 3.3 | 96.1 | 77.5 | 80.6 |
| 4.3 | 94.2 | 76.3 | 81.0 |
| 5.3 | 92.3 | 74.0 | 80.2 |
| 6.3 | 91.0 | 73.1 | 80.3 |

Reference Example 8

ZSM-5 samples (NH$_4$ form) obtained in Reference Example 7, No. 4 (4 g each) were subjected four times, at 90°C for one hour each, to ion-exchange treatment with aqueous 5 % solution (50 ml) of metal salt as (MXn : X = Cℓ$^-$ or OAc$^-$) shown in Table 21. After washing with water, drying and calcining, metal ion-exchanged ZSM-5 were obtained in each case.

Table 21

| Run No. | Metal salt | M·ZSM-5 |
|---|---|---|
| 1 | Ca(OAc)$_2$ | Ca·ZSM-5 |
| 2 | Sr(OAc)$_2$ | Sr·ZSM-5 |
| 3 | LaCℓ$_3$ | La·ZSM-5 |

Example 16 (Test under high WHSV)

Example 1 was repeated except that metal ion-exchanged ZSM-5 samples prepared in Reference Example 8 were used in place of the H·ZSM-5. The results are shown in Tables 22 - 24.

Table 22 (Ca·ZSM-5)

| time elapsed (hr) | cyclohexanone oxime conversion (%) | ε-caprolactam | |
|---|---|---|---|
| | | yield (%) | selectivity (%) |
| 1.3 | 99.1 | 71.4 | 72.0 |
| 2.3 | 97.6 | 73.4 | 75.2 |
| 3.3 | 96.0 | 76.8 | 80.0 |
| 4.3 | 93.8 | 75.1 | 80.1 |
| 5.3 | 92.0 | 73.9 | 80.3 |
| 6.3 | 90.1 | 72.1 | 80.0 |

Table 23 (Sr·ZSM-5)

| time elapsed (hr) | cyclohexanone oxime conversion (%) | ε-caprolactam | |
|---|---|---|---|
| | | yield (%) | selectivity (%) |
| 1.3 | 100 | 75.0 | 75.0 |
| 2.3 | 99.6 | 77.2 | 77.5 |
| 3.3 | 98.3 | 80.1 | 81.5 |
| 4.3 | 97.0 | 79.5 | 82.0 |
| 5.3 | 95.3 | 78.0 | 81.8 |
| 6.3 | 94.0 | 77.4 | 82.3 |

Table 24 (La·ZSM-5)

| time elapsed (hr) | cyclohexanone oxime conversion (%) | ε-caprolactam | |
|---|---|---|---|
| | | yield (%) | selectivity (%) |
| 1.3 | 99.3 | 70.3 | 70.8 |
| 2.3 | 97.0 | 71.0 | 73.2 |
| 3.3 | 95.8 | 75.3 | 78.6 |
| 4.3 | 94.0 | 73.1 | 77.8 |
| 5.3 | 92.5 | 70.8 | 76.5 |
| 6.3 | 90.3 | 69.5 | 77.0 |

Reference Example 9

The following solutions were prepared:

| Solution A | |
|---|---|
| Distilled water | 162 g |
| H$_2$SO$_4$ | 16.7 g |
| Al$_2$(SO$_4$)$_3$ · 17H$_2$O | 0.16 g |
| (n-Pr)$_4$NBr | 20.3 g |

| Solution B | |
|---|---|
| Distilled water | 119.7 g |
| Sodium silicate (JIS No. 3) | 186.3 g |

| Solution C | |
|---|---|
| Distilled water | 281.7 g |
| NaCl | 70.9 g |

Into solution C were dropped at the same time, solutions A and B. The mixture was vigorously stirred, keeping the pH at 9 - 11 with the addition of aqueous 48 % NaOH solution (6 g).

The mixture (pH 9.6) was charged in an autoclave (one ℓ) made of SUS and stirred (≧400 r.p.m.) at 160°C for 20 hours to effect hydrothermal synthesis. After being cooled, the reaction mixture was repeatedly subjected to filtration and washing with a large amount (about 7ℓ) of distilled water until no Cl⁻ was detected. After the mixture was dried at 120°C for 16 hours, the BET external surface area was 18.3 m²/g. The mixture was calcined at 500 - 550°C for 4 hours in an air stream to yield white powdery crystals (48 g) which were identified as ZSM-5 by X ray diffraction.

Ion-exchanging and calcining as in Reference Example 1 were effected to obtain H·ZSM-5.
Surface acidity (pKa) = - 3
4 MQ adsorption amount = 3.62 μ equivalent/g (350°C)
Si/Al atomic ratio = 550

Example 17 (A test under high WHSV)

Example 1 was repeated except the H·ZSM-5 obtained in Reference Example 9 was used in place of the H·ZSM-5. The result is shown in Table 25.

## Table 25

| time elapsed (hr) | cyclohexanone oxime conversion (%) | ε-caprolactam | |
|---|---|---|---|
| | | yield (%) | selectivity (%) |
| 1.3 | 100 | 76.8 | 76.8 |
| 2.3 | 99.3 | 81.0 | 81.6 |
| 3.3 | 98.3 | 79.6 | 81.0 |
| 4.3 | 96.9 | 82.2 | 84.8 |
| 5.3 | 95.4 | 81.0 | 85.0 |
| 6.3 | 93.6 | 79.7 | 85.1 |

17

Comparison Examples 1 - 3 (Tests under high WHSV)

Example 1 was repeated except that H.ZSM-5 samples having various Si/Al atomic ratios (7.9, 17.3 and 49.2) used, respectively, in place of the H•ZSM-5.

The results are shown in Tables 26 - 28.

### Table 26 (Si/Al = 7.9)

| time elapsed (hr) | cyclohexanone oxime conversion (%) | ε-caprolactam | |
|---|---|---|---|
| | | yield (%) | selectivity (%) |
| 1.3 | 60.0 | 26.3 | 43.8 |
| 2.3 | 37.3 | 14.5 | 38.9 |
| 3.3 | 30.5 | 10.1 | 33.1 |

### Table 27 (Si/Al = 17.3)

| time elapsed (hr) | cyclohexanone oxime conversion (%) | ε-caprolactam | |
|---|---|---|---|
| | | yield (%) | selectivity (%) |
| 1.3 | 66.0 | 24.4 | 37.0 |
| 2.3 | 25.8 | 10.1 | 39.1 |
| 3.3 | 8.9 | 4.1 | 46.0 |

### Table 28 (Si/Al = 49.2)

| time elapsed (hr) | cyclohexanone oxime conversion (%) | ε-caprolactam | |
|---|---|---|---|
| | | yield (%) | selectivity (%) |
| 1.3 | 68.5 | 34.3 | 50.1 |
| 2.3 | 24.1 | 13.2 | 53.4 |
| 3.3 | 14.3 | 7.7 | 53.8 |

Example 18

A reactor made of quartz (32 cm long, 1 cm inner diameter) was packed with H•ZSM-5 catalyst (24 - 48 mesh in size, 0.6 g, 1.02 ml) prepared in Reference Example 5, No. 1 and $N_2$ gas was passed therethrough at 350°C for one hour. Cyclohexanone oxime solution (7.53 wt.%) in benzene was charged (WHSV = 9.77 $hr^{-1}$) from a vaporizer. The temperature of the catalyst bed was 350°C.

The result is shown in Table 29.

## Table 29

| time elapsed (hr) | cyclohexanone oxime conversion (%) | ε-caprolactam | |
|---|---|---|---|
| | | yield (%) | selectivity (%) |
| 2 | 100 | 75.0 | 75.0 |
| 3 | 100 | 78.5 | 78.5 |
| 4 | 100 | 77.1 | 77.1 |
| 5 | 100 | 78.7 | 78.7 |
| 6 | 100 | 78.0 | 78.0 |
| 7 | 100 | 79.2 | 79.2 |
| 8 | 100 | 80.6 | 80.6 |
| 9 | 100 | 82.8 | 82.8 |
| 10 | 100 | 81.5 | 81.5 |
| 11 | 100 | 81.2 | 81.2 |
| 12 | 100 | 82.5 | 82.5 |

Example 19

The same reactor as in Example 18 was packed with H·ZSM-5 catalyst (0.6 g, 1.02 ml) prepared in Reference Example 5, No. 1 and $N_2$ gas was passed through at 350°C for one hour. Cyclohexanone oxime solution (7.7 wt.%) in benzene and $CO_2$ gas, a carrier, which had been mixed together were charged (WHSV = 9.83 $hr^{-1}$ and 10 ml/min, respectively) from a vaporizer. The temperature of the catalyst bed was 350°C.

The result is shown in Table 30.

Table 30

| time elapsed (hr) | cyclohexanone oxime conversion (%) | ε-caprolactam | |
| --- | --- | --- | --- |
| | | yield (%) | selectivity (%) |
| 2.5 | 100 | 81.5 | 81.5 |
| 3.5 | 100 | 83.5 | 83.5 |
| 4.5 | 100 | 83.8 | 83.8 |
| 5.5 | 100 | 86.3 | 86.3 |
| 6.5 | 100 | 84.4 | 84.4 |
| 7.5 | 100 | 85.1 | 85.1 |
| 8.5 | 100 | 84.8 | 84.8 |
| 9.5 | 100 | 86.7 | 86.7 |
| 10.5 | 100 | 87.4 | 87.4 |
| 11.5 | 100 | 85.4 | 85.4 |
| 13.5 | 100 | 86.1 | 86.1 |
| 15.5 | 99.5 | 86.7 | 87.1 |
| 17.5 | 98.9 | 86.2 | 87.1 |
| 19.5 | 98.4 | 83.5 | 84.9 |
| 21.5 | 97.2 | 85.0 | 87.5 |
| 23.0 | 96.7 | 85.4 | 86.1 |

Extrapolation of data in Table 2 shows that half value period is about 150 hours.

Example 20

Example 19 was repeated except that $N_2$ gas, a carrier, was employed in place of $CO_2$, and the WHSV was 9.82 $hr^{-1}$ in place of 9.83 $hr^{-1}$.

The result is shown in Table 31.

Table 31

| time elapsed (hr) | cyclohexanone oxime conversion (%) | ε-caprolactam | |
|---|---|---|---|
| | | yield (%) | selectivity (%) |
| 1.3 | 100 | 75.9 | 75.9 |
| 2.3 | 100 | 76.8 | 76.8 |
| 3.3 | 100 | 77.8 | 77.8 |
| 4.3 | 100 | 78.4 | 78.4 |
| 5.3 | 100 | 81.1 | 81.1 |
| 7.3 | 100 | 80.2 | 80.2 |
| 8.5 | 100 | 83.2 | 83.2 |
| 9.5 | 100 | 84.0 | 84.0 |
| 10.5 | 100 | 82.8 | 82.8 |
| 11.5 | 100 | 82.2 | 82.2 |
| 13.5 | 100 | 82.0 | 82.0 |
| 14.5 | 100 | 82.5 | 82.5 |

Example 21

The same reactor as in Example 18 was packed with H·ZSM-5 catalyst (0.6 g, 1.02 ml) prepared in Reference Example 7, No. 1 and $N_2$ gas was passed through at 350°C for one hour. Cyclohexanone oxime solution (8.0 wt.%) in benzene was charged (WHSV = 10.8 $hr^{-1}$) from a vaporizer. The temperature of a catalyst bed was 350°C.

The result is shown in Table 32.

## Table 32

| time elapsed (hr) | cyclohexanone oxmie conversion (%) | ε-caprolactam | |
|---|---|---|---|
| | | yield (%) | selectivity (%) |
| 1.3 | 100 | 73.4 | 73.4 |
| 2.3 | 100 | 76.7 | 76.7 |
| 3.3 | 100 | 79.7 | 79.7 |
| 4.3 | 100 | 81.0 | 81.0 |
| 5.3 | 100 | 83.9 | 83.9 |
| 6.3 | 100 | 84.2 | 84.0 |
| 8.0 | 100 | 86.6 | 86.6 |
| 9.0 | 100 | 88.8 | 88.8 |
| 10.0 | 100 | 87.2 | 87.2 |
| 11.0 | 100 | 86.5 | 86.5 |
| 12.0 | 100 | 86.1 | 86.1 |
| 13.0 | 100 | 85.9 | 85.9 |
| 14.0 | 100 | 88.8 | 88.8 |

Example 22

Example 21 was repeated except that, in place of stopping the reaction after 14 hours, the reaction was continued thereafter by charging $CO_2$ gas, a carrier, (10 ml/min) after 14.5 hours had elapsed since the reaction began.

The result is shown in Table 33.

Table 33

| time elapsed (hr) | cyclohexanone oxime conversion (%) | ε-caprolactam | |
|---|---|---|---|
| | | yield (%) | selectivity (%) |
| 15.5 | 100 | 90.5 | 90.5 |
| 16.5 | 100 | 90.0 | 90.0 |
| 17.5 | 100 | 91.6 | 91.6 |
| 18.3 | 99.6 | 90.2 | 90.6 |
| 19.3 | 99.5 | 90.1 | 90.6 |
| 20.3 | 99.3 | 89.9 | 90.5 |
| 23.0 | 98.6 | 88.7 | 90.0 |
| 24.0 | 97.7 | 89.2 | 91.3 |
| 25.0 | 97.3 | 86.8 | 89.2 |
| 26.0 | 96.5 | 87.9 | 91.1 |
| 27.0 | 95.6 | 86.0 | 90.0 |
| 28.0 | 95.0 | 85.7 | 90.2 |
| 29.0 | 93.9 | 86.2 | 91.9 |

Extrapolation of the conversion rate shows that half value period of catalyst activity, i.e. the period during which the conversion rate decreases to 50%, is about 120 hours.

Example 23

Example 21 was repeated except that H·ZSM-5 prepared in Reference example 7, No. 4 was used in place of the H·ZSM-5.

The result is shown in Table 34.

Table 34

| time elapsed (hr) | cyclohexanone oxime conversion (%) | ε -caprolactam | |
| --- | --- | --- | --- |
| | | yield (%) | selectivity (%) |
| 0.3-1.3 | 100 | 81.1 | 81.1 |
| 2.3 | 100 | 85.5 | 85.5 |
| 3.3 | 99.6 | 85.5 | 85.9 |
| 4.3 | 99.2 | 86.0 | 86.7 |
| 5.3 | 98.7 | 85.7 | 86.9 |
| 6.3 | 98.0 | 83.8 | 85.5 |
| 7.3 | 97.1 | 85.3 | 87.9 |
| 8.3 | 96.4 | 82.5 | 85.6 |
| 9.3 | 95.7 | 82.4 | 86.1 |
| 10.3 | 94.9 | 80.9 | 85.3 |
| 11.3 | 94.2 | 81.0 | 86.0 |
| 12.3 | 93.5 | 80.6 | 86.2 |
| 13.3 | 92.7 | 79.4 | 85.7 |
| 14.3 | 92.0 | 78.4 | 85.2 |
| 15.3 | 91.1 | 78.4 | 86.1 |
| 16.3 | 90.3 | 78.4 | 86.8 |

Comparison Example 4

Example 21 was repeated except that H•ZSM-5 whose Si/Al atomic ratio was 49.2 was used in place of the H•ZSM-5.

The result is shown in Table 35.

Table 35

| time elapsed (hr) | cyclohexanone oxime conversion (%) | ε -caprolactam | |
| --- | --- | --- | --- |
| | | yield (%) | selectivity (%) |
| 1.3 | 80.5 | 42.7 | 53.0 |
| 2.3 | 75.0 | 43.6 | 58.2 |
| 3.3 | 68.7 | 42.0 | 61.1 |
| 4.3 | 62.5 | 40.0 | 64.0 |
| 5.3 | 55.2 | 36.2 | 65.5 |
| 6.3 | 47.3 | 31.1 | 65.7 |

Comparison Example 5

Example 18 was repeated except that silica-alumina catalyst (0.6 g, alumina 26%, manufactured by Shokubai Kasei Co. Ltd.) was used in place of the H•ZSM-5.

The result is shown in Table 36.

## Table 36

| time elapsed (hr) | cyclohexanone oxime conversion (%) | ε -caprolactam | |
|---|---|---|---|
| | | yield (%) | selectivity (%) |
| 2 | 100 | 56.1 | 56.1 |
| 3 | 100 | 58.9 | 58.9 |
| 4 | 100 | 57.5 | 57.5 |
| 5 | 100 | 58.9 | 58.9 |
| 6 | 100 | 61.0 | 61.0 |
| 7 | 100 | 61.6 | 61.6 |
| 8 | 100 | 61.3 | 61.3 |
| 9 | 100 | 64.2 | 64.2 |
| 10 | 100 | 61.6 | 61.6 |
| 11 | 100 | 65.1 | 65.1 |
| 12 | 100 | 64.8 | 64.8 |

Comparison Example 6

Example 18 was repeated except $B_2O_3$/ZnO catalyst (30/70 wt.%, 0.6 g) was used in place of the H•ZSM-5.

The result is shown in Table 37.

## Table 37

| time elapsed (hr) | cyclohexanone oxime conversion (%) | ε-caprolactam | |
|---|---|---|---|
| | | yield (%) | selectivity (%) |
| 2 | 100 | 98.0 | 98.0 |
| 3 | 97.5 | 92.8 | 95.2 |
| 4 | 95.1 | 85.9 | 90.3 |
| 5 | 92.0 | 81.4 | 88.5 |
| 6 | 88.4 | 76.9 | 87.0 |
| 7 | 81.7 | 67.5 | 82.6 |
| 8 | 74.3 | 59.0 | 79.4 |
| 9 | 65.9 | 49.6 | 75.3 |
| 10 | 54.1 | 39.2 | 72.5 |
| 11 | 47.6 | 31.4 | 66.0 |
| 12 | 35.2 | 20.8 | 59.3 |

## Claims

1. A process for preparing ε-caprolactam, by bringing gaseous cyclohexanone oxime into contact with a crystalline alumino-silicate catalyst having a constraint index of from 1 - 12, characterised in that the catalyst has an Si/Al atomic ratio of 500 or more and an external acid functionality of no more than 5 micro equivalent/g.

2. A process according to Claim 1 wherein the alumino- silicate has an external surface area of 5 $m^2$/g or more.

3. A process according to Claim 1 or 2 wherein the alumino-silicate is ZSM-5, ZSM-11, ZSM-23 or Silicalite.

4. A process according to Claim 1 or 2 wherein the Si/Al atomic ratio of the alumino-silicate is 1000 or more.

5. A process according to any one of Claims 1 to 4, wherein external acid functionality of the alumino-silicate is 2 micro equivalent/g or less.

6. A process according to any one of Claims 1 to 5, wherein the alumino-silicate is in $H^+$ form.

7. A process according to any one of Claims 1 to 5, wherein the alumino-silicate is one which has been ion-exchanged with alkaline-earth metal or lanthanide metal.

8. A process according to any one of Claims 1 to 7, wherein the cyclohexanone oxime is diluted with benzene or toluene.

9. A process according to any one of Claims 1 to 8, wherein the cyclohexanone oxime is fed at such a rate that the weight hourly space velocity (WHSV) is from 1 to 50 $hr^{-1}$.

10. A process according to Claim 9 wherein the WHSV is from 5 to 40 $hr^{-1}$.

EP 0 234 088 B1

**11.** A process according to any one of Claims 1 to 10, wherein the cyclohexanone oxime is fed with inert gas as a carrier.

**12.** A process according to any one of Claims 1 to 11, wherein the contact is made at a temperature of 300 - 450ºC.

## Revendications

**1.** Procédé de préparation d'ε-caprolactame, en mettant du cyclohexanone oxime gazeux en contact avec un catalyseur à base d'aluminosilicate cristallin ayant un indice de contrainte de 1 à 12, caractérisé en ce que le catalyseur a un rapport atomique Si/Al de 500 ou supérieur à 500 et une fonctionnalité acide extérieure qui n'est pas supérieure à 5 micro-équivalent/g.

**2.** Procédé suivant la revendication 1, dans lequel l'aluminosilicate a une surface spécifique extérieure de 5 m$^2$/g ou supérieure à 5 m$^2$/g.

**3.** Procédé suivant la revendication 1 ou 2, dans lequel l'aluminosilicate est ZSM-5, ZSM-11, ZSM-23 ou du silicalite.

**4.** Procédé suivant la revendication 1 ou 2, dans lequel le rapport atomique Si/Al de l'aluminosilicate est de 1000 ou est supérieur à 1000.

**5.** Procédé suivant l'une quelconque des revendications 1 à 4, dans lequel la fonctionnalité acide extérieure de l'aluminosilicate est de 2 micro-équivalent/g ou est inférieure à 2 micro-équivalent/g.

**6.** Procédé suivant l'une quelconque des revendications 1 à 5, dans lequel l'aluminosilicate est sous la forme H$^+$.

**7.** Procédé suivant l'une quelconque des revendications 1 à 5, dans lequel l'aluminosilicate est un aluminosilicate dont les ions ont été échangés par un métal alcalino-terreux ou par un métal de lanthanide.

**8.** Procédé suivant l'une quelconque des revendications 1 à 7, dans lequel le cyclohexanone oxime est dilué par du benzène ou par toluène.

**9.** Procédé suivant l'une quelconque des revendications 1 à 8, dans lequel le cyclohexanone oxime est chargé à une vitesse telle que la vitesse spatiale horaire pondérale (VSHP) est comprise entre 1 et 50 h$^{-1}$.

**10.** Procédé suivant la revendication 9, dans lequel la VSHP est comprise entre 5 et 40 h$^{-1}$.

**11.** Procédé suivant l'une quelconque des revendications 1 à 10, dans lequel le cyclohexanone oxime est chargé avec du gaz inerte servant de gaz porteur.

**12.** Procédé suivant l'une quelconque des revendications 1 à 11, dans lequel le contact est effectué à une température de 300 à 450˚ C.

## Patentansprüche

**1.** Verfahren zur Herstellung von ε-Caprolactam, durch Inkontaktbringen von gasförmigem Cyclohexanonoxim mit einem kristallinen Aluminosilikatkatalysator mit einem Constraint Index von 1 bis 12, **dadurch gekennzeichnet**, daß der Katalysator ein Si/Al-Atomverhältnis von 500 oder mehr und eine äußere Säurefunktionalität von nicht mehr als 5 Mikroäquivalenten/g besitzt.

**2.** Verfahren nach Anspruch 1, wobei das Aluminosilikat eine äußere Oberfläche von 5 m$^2$/g oder mehr besitzt.

**3.** Verfahren nach Anspruch 1 oder 2, wobei das Aluminosilikat ZSM-5, ZSM-11, ZSM-23 oder Silikalit ist.

27

4. Verfahren nach Anspruch 1 oder 2, wobei das Si/Al-Atomverhältnis des Aluminosilikats 1000 oder mehr beträgt.

5. Verfahren nach einem der Ansprüche 1 bis 4, wobei die äußere Säurefunktionalität des Aluminosilikats 2 Mikroäquivalente/g oder weniger beträgt.

6. Verfahren nach einem der Ansprüche 1 bis 5, wobei das Aluminosilikat in der H$^+$-Form vorliegt.

7. Verfahren nach einem der Ansprüche 1 bis 5, wobei das Aluminosilikat einem Ionenaustausch mit Erdalkalimetall oder Lanthanidmetall unterworfen wurde.

8. Verfahren nach einem der Ansprüche 1 bis 7, wobei das Cyclohexanonoxim mit Benzol oder Toluol verdünnt wird.

9. Verfahren nach einem der Ansprüche 1 bis 8, wobei das Cyclohexanonoxim in einer derartigen Rate eingegeben wird, daß die Gewicht-Stunden-Raumgeschwindigkeit (weight hourly space velocity, WHSV) 1 bis 50 h$^{-1}$ beträgt.

10. Verfahren nach Anspruch 9, wobei die WHSV von 5 bis 40 h$^{-1}$ beträgt.

11. Verfahren nach einem der Ansprüche 1 bis 10, wobei das Cyclohexanonoxim mit Inertgas als Träger eingegeben wird.

12. Verfahren nach einem der Ansprüche 1 bis 11, wobei der Kontakt bei einer Temperatur von 300 bis 450 °C erfolgt.

28